Europäisches Patentamt

European Patent Office

Office européen des brevets

(11) Publication number: **0 131 783**
**B1**

(12) **EUROPEAN PATENT SPECIFICATION**

(45) Date of publication of patent specification: **13.09.89**

(51) Int. Cl.⁴: **A 01 N 25/26,** A 01 N 25/18,
A 61 L 9/04

(21) Application number: **84107132.7**

(22) Date of filing: **20.06.84**

(54) A sustainedly vapor-releasing body for environmental control.

(30) Priority: **20.06.83 JP 110605/83**
**08.07.83 JP 124161/83**

(43) Date of publication of application:
**23.01.85 Bulletin 85/04**

(45) Publication of the grant of the patent:
**13.09.89 Bulletin 89/37**

(84) Designated Contracting States:
**DE GB IT**

(56) References cited:
**GB-A-1 517 410**
**GB-A-2 067 406**

**MANUFACTURING CHEMIST & AEROSOL
NEWS, vol. 45, no. 4, April 1974, pages 19-21,
London, GB; J.F. PICKARD et al.: "Film coating:
1 Formulation and process considerations"**

(73) Proprietor: **Shin-Etsu Chemical Co., Ltd.**
**6-1, Ohtemachi 2-chome**
**Chiyoda-ku Tokyo 100 (JP)**

(72) Inventor: **Nagura, Shigehiro**
**2-1-4-7, Minato-machi**
**Joetsu-shi Niigata-ken (JP)**
Inventor: **Chiba, Tohru**
**2-14-45, Gochi**
**Joetsu-shi Niigata-ken (JP)**
Inventor: **Niyomura, Katuya**
**150-1, Kuniga**
**Arai-shi Niigata-ken (JP)**
Inventor: **Ogawa, Kinya**
**1-7-32, Shiboku Miyamae-ku**
**Kawasaki-shi Kanagawa-ken (JP)**
Inventor: **Aiba, Noboru**
**3-2-21, Kitamoto-cho**
**Joetsu-shi Niigata-ken (JP)**
Inventor: **Yamamoto, Akira**
**3-8-11, Higashishiro-machi**
**Joetsu-shi Niigata-ken (JP)**
Inventor: **Aizawa, Michio**
**2-15-3-509, Masago**
**Chiba-shi Chiba-ken (JP)**

**EP 0 131 783 B1**

(74) Representative: Jaeger, Klaus, Dipl.-Chem. Dr.
et al
Jaeger, Steffens & Köster Patentanwälte
Pippinplatz 4a
D-8035 München-Gauting (DE)

**Description**

Background of the invention

The present invention relates to a sustainedly vapor-releasing body for environmental control or, more particularly, to a vapor-releasing body capable of emitting a vapor diffusible into and capable of exhibiting activities in the atmosphere at a controlled rate. The inventive vapor-releasing body is used as an emitter of a sex pheromone of noxious insects or pests to provide an efficient means for the extermination or control of the pests in the agriculture and forestry.

In connection with vaporizable or sublimatable substances diffusible into and capable of exhibiting activities in the atmosphere even in an extremely low concentration, such as a sex pheromone of insects and a perfume or deodorant, it is very important that the vapor of the active substance is emitted or released sustainedly at a controlled rate into the atmosphere over a length of time so that various attempts have been proposed for sustainedly controlling the rate of vapor-releasing of such substances. For example, insect sex pheromones are shaped into a form like a solid medicament from which the vapor of the pheromone is emitted at a controlled rate to exhibit the desired effect sustainedly in the atmosphere.

A composition for the sustained release of the vapor of an insect pheromone should satisfy several requirements inclding, for example:

(1) a constant or controlled rate of emission of the pheromone over a desired length of time;

(2) stability of the pheromone contained therein to cause loss thereof as small as possible;

(3) biodegradability of the components to cause no environmental pollution;

(4) easiness of application thereof in the field; and

(5) easiness of preparation and inexpensiveness, while no prior art compositions or bodies hitherto developed can satisfy all of these requirements.

Various types of compositions and bodies for sustained release of pheromones have been proposed in the prior art including microcapsules by use of gelatin or polyamide resins disclosed in U.S. Patents 2,800,457, 2,800,458 and 3,577,515, film dispensers of multi-layered structure disclosed in A.C.S., volume 33, page 283 (1976), hollow fiber capillaries having open ends disclosed in U.S. Patent 4,017,030 and sticking agents admixed with a pheromone compound. These prior art pheromone releasing compositions or bodies have their respective disadvantages and problems. For example, the problems of the microcapsules are that gelatin has poor weathering resistance and polyamide resins are not biodegradable, in addition to the expensiveness. The film dispersers and hollow fibers have a deficiency that, in addition to the problem of the non-biodegradability of the materials forming the films or fibers, they must be distributed in the fields by use of a special distributor machine. The pheromone-admixed sticking agents are defective due to the difficulty in controlling the releasing velocity of the pheromone which is sometimes excessively large directly after application of the sticking agent while the releasing velocity rapidly decreases with lapse of time, consequently with unsatisfactorily short service life. This deficiency of more remarkable in hot weather as in summer.

Furthermore, in GB 2 067 406 A there has been proposed a powdery formulation, wherein the pheromone is supported on an inert carrier material, admixed with an organic resinous binder and coated with a film-forming resin. The disadvantages of this composition are again the poor biodegradability of the binder or the coating materials and the relative low overall contents regarding the active substance.

Another important class of the compounds for which a means for sustainedly contrclling the rate of vapor emission to the atmosphere is desired is perfumery since perfumes as well as deodorants are usually vaporizable and the pleasant effect obtained with a perfume is naturally transient because the effect is obtained only by the vapor of a perfume floating in the air in a trace concentration.

Various methods have been proposed for sustainedly obtaining the effect of a perfume without frequently repeating spraying of a liquid perfume including a method in which a solid base material such as a gum, silica gel, active charcoal and the like is impregnated with the perfume and emission of the perfume takes place from the thus perfume-impregnated solid at room temperature or with heating, a method in which the perfume is contained and confined in a container having a small opening so that the emission of the perfume to the atmospheric air takes place only through the opening or a method in which a sublimitable substance is impregnated with the perfume and the emission of the perfume takes place along with the sublimation of the sublimatable substance.

These conventional methods for the sustained release of perfumes are not always quite satisfactory, for example, because of the requirement for a specific device and unstable rate or insufficiently long duration of the perfume emission. In addition, these conventional methods are not applicable when a sustainedly perfume-releasing body in a particular or granular form is desired to facilitate handling.

Recently, some proposals have been made for sustainedly controlling the emission of perfumes including a granular perfume-dispenser prepared by providing a coating film of a vapor-permeable cellulose derivative on the granules of an active alumina impregnated with a perfume (see, for example, Japanese Patent Publication 56-7423) and a perfumery tablet prepared by the adsorption of a perfume on a hygroscopic adsorbent followed by tableting and coating of the tablet with a polymeric coating material in such a thickness that the perfume can be released to the atmosphere only when the tablet is heated (see, for example, Japanese Patent Kokai 53-69840). These methods are also not free from some problems that the rate of perfume releasing from the granular perfume dispenser is relatively large in the beginning stage of its use but rapidly decreases in the lapse of time not to provide good control of the releasing rate in addition to the relatively large loss of the perfume in the preparation of the perfumery tablet which is effective only when it is heated.

The above mentioned sex pheromones of insects belong only to a part of typical classes of compounds gasifiable by vaporization or sublimation and diffusible into the atmosphere of which efficient means for sustained and controlled release of the vapor are desired including deodorants, insecticides, bactericides, pest repellents and fumigants. All of the sustainedly vapor-releasing bodies for these substances should satisfy similar requirements to those in the insect sex pheromones while none of the prior art materials is quite satisfactory in all respects. Similarly to pheromones, for example, the biodegradability of the components to cause no environmental pollution and the easiness in the application to the fields are some of the essential conditions in the sustainedly vapor-releasing bodies for insecticides, bactericides and pest repellents used in forestry, agriculture and horticulture.

Summary of the invention

It is therefore the object of the present invention to provide a sustainedly vapor-releasing body for emission of a sex pheromone compound of insects at a controlled, constant rate over a desired time period consisting of components causing no environmental pollution, and which is easy to distribute in the outdoor area of application, easy to prepare and inexpensive.

The sustainedly vapor-releasing body of the present invention developed as a result of the expensive investigations continued by the inventors comprises a solid granulated particle formed of a composition comprising a sex pheromone compound of insects capable of exhibiting an activity when contained in the atmospheric air in an extremely low concentration, an inert carrier material for the gasifiable and diffusible substance and a binder and a coating layer on the surface of the solid particle formed of a polymeric material. In particular, the coating film of a polymeric material may contain an inorganic powdery material in a specified proportion to the polymeric base material.

Detailed description of the preferred embodiment

The above described sustainedly vapor-releasing body of the present invention can satisfy all of the requirements for such materials described in (1) to (5) above. Moreover, it is a relatively easy matter to adequately control the velocity of emission of the active substance from the inventive sustainedly vapor releasing body by the suitable selection and adjustment of the relative amounts of the inert carrier material and the binder, the particle size or surface area of the body and the thickness of the coating layer as well as by the incorporation of an inorganic powdery material into the coating layer. In addition, the sustainedly vapor-releasing body of the invention can be a tiny particle of any small size suitable for the preparation by a conventional method of granulation so that the particles can be distributed over the fields in the form of the granules as such or in the form of an aqueous suspension containing a spreading agent, according to need, by use of an ordinary distributor machine without consumption of a large manpower in the case of sex pheromones of insects, insecticides or bactericides used in agriculture and horticulture. In the case of pheromone-containing vapor-releasing bodies, in particular, the invention provides a possibility of obtaining a more uniform distribution of the pheromone-emitting source over the field in the application for disturbing the intercommunication of insects with sustained release of the pheromone at a constant and controlled rate to achieve more efficient utilization of the pheromone. An additional advantage of the present invention is the degradability of the base materials used in the vapor-releasing body after a certain time without the danger of accumulation in nature to cause environmental pollution.

The active ingredient in the inventive sustainedly vapor-releasing body is a vaporizable and diffusible subsance capable of exhibiting certain activity when it is gasified by vaporization or sublimation and contained in the atmospheric air even in an extremely low concentration. Examples of such a material are sex pheromones of insects.

The above mentioned insect pheromone belonging to an important class of the gasifiable and diffusible active substances is not particularly limited to a specific type but may be any one capable of exhibiting the desired activity to the species of the insects to be controlled.

The pheromone may be a single compound or a mixture of a plurality of active compounds. It is also optional that the pheromone is admixed with conventional additives such as a stabilizer typically represented by ultraviolet absorbers and antioxidants.

The solid particle is formed of a composition comprising the gasifiable and diffusible active substance, an inert carrier material and a binder.

The material suitable as a carrier of the active substance i.e., insect pheromones should be inert to the active substance and have good capacity of absorbing the active substance. Such an inert carrier material is anhydrous silicic acid, and derivatives thereof in a powdery form. The weight proportion of the active insect pheromone component to the inert carrier material of course depends on the type of the active component and the intended use of the inventive sustainedly vapor-releasing body but it is usually in the range from 1:0.3 to 1:10 or, preferably is from 1:0.3 to 1:3 when the active component is an insect pheromone.

The binder may be selected from the group of cellulose derivatives. It is advantageous to use a binder material having good miscibility or compatibility with the active component i.e. the insect pheromone.

The coating layer provided on the surface of the solid particles containing the gasifiable and diffusible

4

active substance is formed of a film-forming polymeric material having an adequate permeability to the vapor of the gasifiable active substance contained in the solid particle such as those named above as the examples of the binder material. It is also important that the film-forming polymeric material for the coating layer has a good degradability by bacteria or enzymes so as not to be accumulated in nature without causing the problem of environmental pollution. The film-forming polymeric material should desirably be soluble in a solvent having a boiling point of 120°C or below. These film-forming polymeric materials may be used either singly or as a combination of two kinds or more according to need provided that they have good compatibility with each other. In particular, the cellulose derivatives are exemplified by methyl cellulose, hydroxyethyl cellulose, hydroxypropyl cellulose, hydroxyethyl methyl cellulose, hydroxypropyl methyl cellulose, ethyl cellulose, ethyl carboxymethyl cellulose, carboxymethyl cellulose or other cellulose ethers, cellulose acetate, or cellulose acetate phthalate, cellulose esters and hydroxypropyl methyl cellulose phthalate, hydroxypropyl methyl cellulose acetate succinate or cellulose ether esters.

These cellulose derivatives are very satisfactory as the coating material used in the inventive sustainedly vapor-releasing body because they have a good film-forming property to coat the solid particles with a film having an adequate permeability to the gasifiable and diffusible active substance to provide a means of controlling the rate of emission of the active substance into the atmospheric air. In addition, these cellulose derivatives are readily decomposed by bacteria or cellulase to be returned to nature without causing the problem of environmental pollution.

When the cellulose derivative used for coating the solid particles impregnated with the gasifiable and diffusible active substance is insoluble in water having a value of pH at or in the vicinity of 7, the sustainedly vapor-releasing body of the invention is stable even in contact with water. When the cellulose derivative is soluble in water, on the other hand, the sustainedly vapor-releasing body of the invention can no longer be stable in contact with water. It is preferable in such a case, therefore, that the sustainedly vapor-releasing body coated with the cellulose derivative is subjected to a treatment for insolubilizing the cellulose derivative against water when the vapor-releasing body is expected to be used with a possibility of being brought into contact with water although such an insolubilizing treatment of the cellulose derivative is not important for the vapor-releasing body used without being brought into contact with water. Generally, sustainedly vapor-releasing bodies for the emission of a sex pheromone of insects are used in an open field where the vapor-releasing bodies are frequently wet with rain or water sprinkling so that the insolubility of the cellulose derivative is very important in order to avoid dissolution of the coating film and disintegration of the body.

The coating film on the solid particles may not be formed of a single kind of the cellulose derivatives but is optional to use two or more kinds of the cellulose derivatives in combination according to need to provide the coating film on the solid particles. It is also optional that the cellulose derivative is admixed with a wax-like material such as stearic acid which serves as a film-forming aid. The coating layer may be formed of a blend of the above mentioned film-forming organic polymer and an inorganic powdery material. Such an inorganic powdery material may be anhydrous silicic acid, various kinds of silicates and talc and serve to further facilitate control of the releasing rate of the active substance contained in the solid particles. The amount of the inorganic powdery material in the coating layer should be 4 times by weight or less of the film-forming polymeric material.

A typical formulation of the inventive sustainedly vapor-releasing body is as follows, in which the amount of each of the components is given in % by weight based on the total weight of the body.

Base particles:
| | |
|---|---|
| Active ingredient | 0.5 to 60% by weight |
| Stabilizer | 0 to 6% by weight |
| Inert carrier | 10 to 50% by weight |
| Binder | 1 to 30% by weight |

Coating layer:
| | |
|---|---|
| Film-forming polymer | 5 to 50% by weight |
| Inorganic powder | 0 to 30% by weight |

The above given formulation should of course be within the preferable limitations that the weight ratio of the active ingredient, e.g. insect pheromone to the inert carrier is in the range from 1:0.3 to 1:3, the weight ratio of the inert carrier to the binder is in the range from 1:0.1 to 1:3 and the weight ratio of the film-forming polymeric material to the inorganic powdery material is in the range from 1:0 to 1:4. When the active ingredient is an insect pheromone, in particular, it is preferable that the content thereof is at least 11% by weight. When the content of the active ingredient is lower than the above mentioned range, the rate of vapor emission thereof from the body may be too low so that no sufficiently high activity of the active substance can be exhibited in the atmosphere. For example, the vapor-releasing body containing an insect pheromone in such a low content must be distributed in the field in an excessively large amount which leads to an economical disadvantage and decreased adherence to the twigs of trees when the vapor-releasing bodies are distributed by the aid of a spreading agent. Higher contents of the active ingredient over 60% by weight is disadvantageous due to the increased vaporization loss in the course of the preparation of the inventive vapor-releasing body as well as the difficulty in the control of the rate of vapor emission.

The typical procedure for the preparation of the sustainedly vapor-releasing body of the invention is as follows.

In the first place, the active ingredient such as an insect pheromone, an inert carrier material and, if necessary, stabilizer for the active ingredient are introduced into a suitable blending machine and blended together so that the active ingredient is adsorbed on the inert carrier material. It is optional in this case that a small volume of a suitable organic solvent is added in order to accelerate uniform blending. Thereafter, a solution of the binder is added and thoroughly mixed or, alternatively, a binder in a powdery form is added and mixed together followed by the admixture of a solvent for the binder to continue blending. The thus obtained mixture of the active ingredient, inert carrier, binder and, optionally, stabilizer is then granulated into granules or balls having an average diameter of 0.3 to 10 mm by use of a suitable granulator.

The solvent for the binder can preferably dissolve also the active ingredient, since otherwise a drawback may be that the active ingredient is isolated on the surface of the particles.

The particles or spherical bodies containing the active ingredient are then provided with a coating film of the film-forming polymeric material, optionally, admixed with an inorganic powdery material. The method for coating is well known in the art including (a) a method in which the solid particles are brought into the fluidized state and a solution of the film-forming polymeric material, optionally, containing the inorganic powdery material in suspension is sprayed onto the particles and (b) a method in which the solid particles are admixed with a solution of the film-forming polymeric material followed by the admixture of the inorganic powdery material and dividing the mixture into a particulate form which is then dried in a fluidized state, if necessary.

In the above mentioned method (a), the coating procedure on the fluidized particles should preferably be conducted under control of the temperature, for example, in the range from 5 to 50°C since a higher temperature may cause an increased vaporization loss of the active ingredient while a lower temperature is undesirable due to the extremely slow evaporation of the solvent to cause sticking of the particles together without giving a uniform coating. It is noted that the solvent for the film-forming polymeric material should have a boiling point of 120°C or below because the velocity of evaporation of a solvent boiling above 120°C is too low so that frequent sticking of the particles is caused which can be avoided only by increasing the temperature of the base particles above 50°C with the sacrifice of the increased vaporization loss of the active ingredient.

The concentration of the film-forming polymeric material in the solution under spraying on to the base particles is preferably in the range from 0.5 to 10% by weight and the solution may contain the inorganic powdery material in suspension in an amount within the above described limit. The addition of the inorganic powdery material is advantageous not only in easing the control of the rate of vapor emission from the finished vapor-releasing body, but also in the prevention of sticking of the particles together in the course of the spray coating to improve the uniformity of coating and in the possibility of decreasing the temperature of the particles under the procedure of coating. Similar advantages are obtained by the admixture of the inorganic powdery material also in the method (b) described above.

As a modification of the spray coating of the core particles, it is optional that the particles are suspended or dispersed in a solution of the film-forming polymeric material and the dispersion or suspension is spray-dried to form dried particles coated with the film of the film-forming polymeric material.

The coating amount of the film-forming polymeric material such as the cellulose derivatives on the solid base particles should usually be in the range from 5 to 70% by weight based on the weight of the base particles. The emission velocity of the gasifiable and diffusible active substance from the sustainedly vapor-releasing body can be controlled in a wide range by suitably selecting various parameters such as the weight proportion of the gasifiable and diffusible active substance and the inert carrier material, surface area of the base particles, type of the film-forming polymeric material and thickness of the coating film formed of the film-forming polymeric material.

In the following, the sustainedly vapor-releasing body of the present invention is described in more detail by way of examples.

Example 1

Spherical or cylindrical base beads of about 1 mm diameter were prepared of a uniform blend composed of 30 parts by weight of a light anhydrous silicic acid (Aerosil® a product by Nippon Aerosil Co.) and 30 parts by weight of cis-11-tetradecenyl acetate known as a sex pheromone of a noxious insect "Tea toatrix" with admixture of 2.0 parts by weight of a hydroxypropyl cellulose (HPC, a product by Shin-Etsu Chemical Co.) as a binder and 180 parts by weight of ethyl alcohol as a wetting agent by use of a conventional extrusion type apparatus for beads preparation.

After drying, the thus prepared base beads were provided with a coating film of a hydroxypropyl methyl cellulose phthalate (HP-55, a product by Shin-Etsu Chemical Co.) in a coating amount of 30% by weight based on the base beads by spraying a 6% solution of the cellulose derivative in a 8:2 by weight mixture of ethyl alcohol and water by use of a fluidizing coater.

The content of the active ingredient cis-11-tetradecenyl acetate in the thus coated beads was determined by distintegrating the beads and extracting the ingredient in acetone and analyzing the acetone extract by the gas chromatography using methyl laurate as an internal standard to give a result of 36.7% by weight based on the amount of the coated beads. The rate of emission of the active ingredient from the thus coated beads was examined by keeping 270 mg of the coated beads containing about 100 mg of the active ingredient under an air flow of 0.5 m/second at 30°C and periodically determining the weight decrease along with the gas chromatographic analysis of the ingredient to give the results shown in Table 1 below indicating the remaining amount of the pheromone compound in mg after keeping the beads up to 70 days under the above mentioned conditions.

As is understood from this table, the emission rate of the pheromone compound was approximately constant at about 2.1 mg/day during the first 40 days and the remaining amount of the pheromone compound after 60 days was only 5% or smaller of the initial impregnation indicating that the coated beads here prepared were quite satisfactory as a sustainedly vapor-releasing body for an insect sex pheromone.

Stability of the coated beads against water was examined by dipping the beads in water at 30°C for 10 days to find no noticeable changes in the appearance of the beads and in the pheromone compound contained therein indicating the high stability of the coated beads against water.

Example 2

The procedures for the preparation of the base beads containing cis-11-tetradecenyl acetate and coating thereof with hydroxypropyl methylcellulose phthalate were substantially the same as in Example 1 except that the coating amount of the cellulose derivative was 50% by weight based on the base beads. The analysis of the coated beads undertaken in the same manner as in Example 1 indicated that the content of the pheromone compound therein was 31.5% by weight.

The test of the coated beads for the releasing velocity of the pheromone compound was performed in the same manner as in Example 1 taking 320 mg of the coated beads at the start. The decrease of the pheromone content in the lapse of time was as shown in Table 1 over 70 days of exposure to air. The releasing rate of the pheromone compound was approximately constant and about 1.5 mg/day for the first 50 days.

TABLE 1

| Days of atmos-pheric exposure of beads | Remaining amount of pheromone, mg | | | |
|---|---|---|---|---|
| | Example 1 | Example 2 | Example 4 | Example 5 |
| 0 | 100 | 100 | 100 | 100 |
| 10 | 75 | 81 | 66 | 75 |
| 20 | 55 | 65 | 42 | 56 |
| 30 | 33 | 52 | 24 | 40 |
| 40 | 17 | 38 | 14 | 28 |
| 50 | 8 | 25 | 7 | 18 |
| 60 | 3 | 15 | 2 | 10 |
| 70 | 1 | 9 | | 6 |

Comparison of the results obtained in Examples 1 and 2 indicates that the releasing velocity of the pheromone from the coated beads can be controlled by suitably selecting the coating amount on the base beads.

Example 3

A uniform blend of a light anhydrous silicic acid and cis-11-tetradecenyl acetate in equal amounts was subjected to granulation and coating in a fluidizing apparatus by spraying a 5% by weight solution of a cellulose acetate (L-20, a product by Dia-cel Chemical Co.) in a 9:1 by weight mixture of methylene chloride and ethyl alcohol. The average particle diameter of the granules was about 0.4 mm and the coating amount of the cellulose acetate was 45% by weight based on the base granules.

The thus prepared granules were disintegrated in and extracted with acetone and the acetone extract was analyzed by the gas chromatography to find that the content of cis-11-tetradecenyl acetate in the granules was 23% by weight. The tests for the velocity of pheromone emission from the granules and the stability of the granules against water gave substantially the same results as in Example 1.

7

Example 4

Base beads of spherical and cylindrical forms having an average diameter of 1 mm were prepared by granulating in an extrusion granulator a uniform blend prepared by mixing 30 parts by weight of a light anhydrous silicic acid, 3 parts by weight of a stabilizer for pheromone compounds and 30 parts by weight of a 1:1 by weight mixture of cis-11-hexadecenyl acetate and cis-11-hexadecenyl aldehyde followed by the addition of 180 parts by weight of a 5% solution of the same cellulose acetate as used in Example 3 in a 9:1 by weight mixture of methylene chloride and ethyl alcohol as a binder.

The thus prepared base beads were provided with a coating film of an ethylcellulose (Ethocell Standard®, a product by Dow Chemical Co.) in a coating amount of 20% by weight based on the base beads by spraying a 6% solution of the ethylcellulose in a 8:2 by weight mixture of ethyl alcohol and toluene in a fluidizing coater with warm air at 45°C as the fluidizing medium. The analysis of the thus finished coated beads gave the results that the contents of cis-11-hexadecenyl acetate and cis-11-hexadecenyl aldehyde in the beads were 17.2% by weight and 17.0% by weight, respectively, to give a total content of the pheromone compounds of 34.2% by weight indicating that the loss of the pheromone compounds was negligibly small by the evaporation or denaturation in the course of the preparation of the base beads and coating.

The coated beads were subjected to the test of the releasing velocity of the pheromone compounds in the same manner as in Example 1 taking 292 mg of the beads at the start. The results obtained by the determination of the remaining amount of the pheromone compounds were as shown in Table 1.

Example 5

The procedures for the preparation of the base beads and the coating thereof with the ethylcellulose were the same as in Example 4 except that the coating amount of the ethylcellulose was 40% by weight based on the base beads. The analysis of the coated beads gave the results that the contents of cis-11-hexadecenyl acetate and cis-11-hexadecenyl aldehyde were 14.6% by weight and 14.5% by weight, respectively, to give a total content of the pheromone compounds of 29.1% by weight. The coated beads were subjected to the test of the releasing velocity of the pheromone compounds in the same manner as in Example 1 to give the results shown in Table 1. The results obtained in Examples 4 and 5 leads to a conclusion that the pheromone-containing coated beads according to the invention are quite satisfactory in the sustained vapor releasability of the pheromone compounds.

Example 6

Substantially the same procedures for the preparation of the base beads and coating thereof as in Example 1 were performed with varied combinations of the types and amounts of the pheromone compounds, stabilizer for the pheromone compounds, inert carrier materials, binders, and film-forming polymeric materials as indicated in Table 2 below in which the content of each of the components is given in % by weight. The pheromone-containing coated beads prepared in each of the Examples were quite satisfactory in respect of the sustained releasability of the pheromone vapor.

In Table 2, the names of the pheromone compounds, the binder materials and the film-forming polymeric materials were abridged as follows.

(Pheromone compounds)
Z-11-HDA: cis-11-hexadecenyl acetate
Z-11-HDAL: cis-11-hexadecenyl aldehyde

(Binders and film-forming polymeric materials)
EC: ethylcellulose
CA: cellulose acetate

8

TABLE 2

| Example No. | 6 |
|---|---|
| Pheromone compound (%) | Z-11-HDA (9.5)<br>Z-11-HDAL (9.5) |
| Stabilizer of pheromone, % | 1.9 |
| Inert carrier, (%) | Anhydrous silicic acid (20) |
| Binder (%) | CA (15) |
| Film-forming polymeric material (%) | EC (44) |
| Average particle diameter of finished particles, mm | 0.7 |

Example 7

Substantially the same procedures for the preparation of the base beads and coating thereof as in Example 1 were undertaken except that the coating liquid used in this case was a suspension of 6 parts by weight of talc in 100 parts by weight of a 6% solution of the same hydroxypropyl methylcellulose phthalate in a 8:2 by weight mixture of ethyl alcohol and water and the temperature of the fluidizing air was 35°C instead of 45°C. The coating amount of the mixture of the cellulose derivative and talc was 40% by weight based on the base beads. The coating procedure could be performed with no troubles of sticking together of the beads at all.

Analysis of the thus obtained pheromone-containing coated beads gave a result that the content of the pheromone compound in the beads was 34.6% by weight indicating that almost no loss of the compound had taken place by evaporation in the course of the processing.

The coated beads were subjected to the test of the releasing velocity of pheromone compound in the same manner as in Example 1 by taking 290 mg of the beads at the start to give the results shown in Table 3, from which it was concluded that the releasing velocity of the pheromone was approximately constant and 2.8 mg/day for the first 25 days and the remaining amount of the compound after lapse of 50 days was only about 5% so that the coated beads were very satisfactory in respect of the well controlled releasing velocity and the extremely small eventually remaining and unutilizable amount of the pheromone compound.

Example 8

The same procedures for the preparation of the base beads and coating thereof as in the preceding example were performed except that the coating amount was 66% by weight based on the base beads. The content of cis-11-tetradecenyl acetate in the thus obtained coated beads determined by the gas chromatographic analysis was 29.1% by weight.

The coated beads were subjected to the test of the releasing velocity of the pheromone compound in the same manner as in Exmaple 1 by taking 345 mg of the beads at the start to give the results shown in Table 3.

Example 9

Substantially the same procedures for the preparation of the base beads and coating thereof as in Example 7 were performed except that the suspension of talc as a coating liquid was prepared by dispersing 2 parts by weight of the talc in 100 parts by weight of the 6% solution of the same cellulose derivative in the same solvent mixture and the temperature of the fluidizing air was 39°C instead of 45°C. The coating amount was 50% by weight based on the base beads. The content of cis-11-tetradecenyl acetate in the thus prepared coated beads was 32.0% by weight.

The pheromone-containing coated beads obtained as above were subjected to the test of the releasing velocity of the pheromone compound in the same manner as in Example 1 by taking 310 mg of the beads at the start to give the results shown in Table 3 below, from which it is clear that the releasing velocity in this case is somewhat lower than in Examples 7 and 8 indicating good controllability of the releasing velocity by the formulation and amount of coating.

TABLE 3

| Days of atmos-pheric exposure of beads | Remaining amount of pheromone, mg | | |
|---|---|---|---|
| | Example 7 | Example 8 | Example 9 |
| 0 | 100 | 100 | 100 |
| 10 | 64 | 76 | 80 |
| 20 | 39 | 53 | 61 |
| 30 | 22 | 37 | 44 |
| 40 | 12 | 24 | 32 |
| 50 | 6 | 16 | 22 |
| 60 | | 10 | 14 |
| 70 | | | 7 |

Example 10

Fifty parts by weight of the base beads prepared in Example 7 were mixed with 65 parts by weight of a 20% solution of a hydroxypropyl methylcellulose phthalate in a 8:2 by weight mixture of ethyl alcohol and water and then with 50 parts by weight of talc followed by dividing into granules and drying by fluidization with warm air at 40°C as the fluidizing medium. The analysis of the thus obtained pheromone-containing coated granules performed in the same manner as in Example 1 gave a result that the content of the pheromone compound therein was 21.2% by weight of cis-11-tetradecenyl acetate indicating substantial absence of the evaporation loss of the compound during the processing.

Example 11

Substantially the same procedures for the preparation of the base beads and coating as in Example 1 were performed with varied combinations of the types and amounts of the pheromone compounds, stabilizers of pheromone compounds, inert carriers, binders, film-forming polymeric materials and inorganic powdery materials in the coating film as indicated in Table 4 below. The thus prepared pheromone-containing coated beads in each of the Examples were quite satisfactory as a sustainedly vapor releasing body of the pheromone compound.

The abridgements in Table 4 for the names of the pheromone compounds, binders and film-forming polymeric materials are the same as in Table 2.

TABLE 4

| Example No. | 11 |
|---|---|
| Pheromone compound (%) | Z-11-HDA (12) Z-11-HDAL (12) |
| Stabilizer of pheromone, % | 2.5 |
| Inert carrier, (%) | Anhydrous silicic acid (25) |
| Binder (%) | CA (19) |
| Film-forming polymeric material (%) | EC (20) |
| Inorganic powder (%) | Talc (10) |
| Average particle diameter of finished particles, mm | 1.2 |

## EP 0 131 783 B1

**Claims**

1. A sustainedly vapor-releasing body for emission of an insect sex pheromone compound composed of a solid base particle made of an inert carrier material and of an organic resinous binder, said particle being impregnated by a pheromone and coated by a film-forming polymeric material, through which the sex pheromone compound outwardly diffuses to be emitted to the ambient atmosphere, characterized in that the inert carrier material is a light anhydrous silicic acid and the binder is a cellulose derivative, wherein the weight fractions of the components based on the overall weight of the body are from 11 to 60% of the sex pheromone, from 10 to 50% of the light anhyhdrous silicic acid, from 1 to 30% of the cellulose derivative as a binder and from 5 to 50% of the film-forming polymeric material in the coating layer with the proviso that the weight ratio of the sex pheromone compound to the light anhydrous silicic acid is in the range from 1:0.3 to 1:3, and this composition of the solid base particle is formed to beads having a diameter in the range from 0.3 to 10 mm.

2. The sustainedly vapor-releasing body as claimed in Claim 1 wherein the film-forming composition forming the coating layer on the solid base particle further comprises an inorganic powdery material.

3. The sustainedly vapor-releasing body as claimed in Claim 1 wherein the weight ratio of the coating layer to the solid base particle is in the range from 5 to 70%.

4. The sustainedly vapor-releasing body as claimed in Claim 2 wherein the amount of the inorganic powdery material in the coating layer is 4 times by weight or smaller of the amount of the film-forming polymeric material.

5. The sustainedly vapor-releasing body as claimed in Claim 1 wherein the film-forming polymeric material is a derivative of cellulose soluble in a solvent having a boiling point of 120°C or below.

**Patentansprüche**

1. Andauernd gasfreisetzender Körper zur Emission eines Insekten-Sexualpheromons, wobei dieser Körper aus einem festen Basispartikel aufgebaut ist, das aus einem inerten Trägermaterial und einem organischen harzartigen Bindemittel hergestellt ist, wobei dieses Partikel mit einem Pheromon getränkt und von einem filmbildenden Polymermaterial überzogen ist, durch welches das Sexualpheromon nach außen diffundiert, um an die umgebende Atmosphäre emittiert zu werden, dadurch gekennzeichnet, daß es sich bei dem inerten Trägermaterial um eine leichte wasserfreie Kieselsäure und bei dem Bindemittel um ein Cellulosederivat handelt, wobei das Sexualpheromon 11 bis 60%, die leichte wasserfreie Kieselsäure 10 bis 50%, das Cellulosederivat als Bindemittel 1 bis 30% und das filmbildende Polymermaterial in der Überzugsschicht 5 bis 50% der Gewichtsanteile der Komponenten ausmachen, bezogen auf das Gesamtgewicht des Körpers, mit der Maßgabe, daß das Gewichtsverhältnis des Sexualpheromons zur leichten wasserfreien Kieselsäure im Bereich von 1:0,3 bis 1:3 liegt, und daß diese Zusammensetzung des festen Basispartikels zu Kügelchen mit einem Durchmesser im Bereich von 0,3 bis 10 mm geformt ist.

2. Andauernd gasfreisetzender Körper nach Anspruch 1, bei den filmbildende Zusammensetzung, welche die Überzugsschicht auf dem festen Basispartikel bildet, außerdem ein pulverförmiges anorganisches Material aufweist.

3. Andauernd gasfreisetzender Körper nach Anspruch 1, bei dem das Gewichtsverhältnis der Überzugsschicht zum festen Basispartikel im· Bereich von 5 bis 70% liegt.

4. Andauernd gasfreisetzender Körper nach Anspruch 2, bei dem die Menge an pulverförmigem anorganischem Material in der Überzugsschicht das vierfache oder weniger, bezogen auf das Gewicht, der Menge des filmbildenden Polymermaterials ausmacht.

5. Andauernd gasfreisetzender Körper nach Anspruch 1, bei dem das filmbildende Polymermaterial ein in einem Lösungsmittel mit einem Siedepunkt von 120°C oder darunter lösliches Cellulosederivat ist.

**Revendications**

1. Corps à libération progressive de vapeurs, en vue de l'émission d'un composé d'une phéromone sexuelle d'insectes comprenant une particule de base solide constituée d'un matériau porteur inerte et d'un liant résineux organique, ladite particule étant imprégnée d'une phéromone et recouverte d'un matériau polymère formant une pellicule, au travers de laquelle le composé de phéromone sexuelle diffuse vers l'extérieur pour être émis vers l'atmosphère ambiante, caractérisé en ce que le matériau porteur inerte est un acide silicique anhyde léger, et le liant est un dérivé de la cellulose dans laquel les fractions pondérales des composants basées sur le poids total du corps sont de 11 à 60% de la phéromone sexuelle; de 10 à 50% de l'acide silicique anhydre léger, de 1 à 30% du dérivé de la cellulose en tant que liant, et de 5 à 50% du matériau polymère formant une pellicule dans la couche de revêtement avec la condition que le rapport pondéral du composé de la phéromone sexuelle à l'acide silicique anhydre léger est compris entre 1:0,3 et 1:3 et cette composition de la particule de base solide est réalisée en cordons ayant un diamètre compris entre 0,3 et 10 mm.

2. Corps à libération progressive de vapeurs selon la revendication 1 dans lequel la composition

11

formant la pellicule constituant la couche de revêtement sur la particule de base solide comprend en outre un matériau inorganique pulvérulent.

3. Corps à libération progressive de vapeurs selon la revendication 1 dans lequel le rapport en poids de la couche de revêtement à la particule de base solide est compris entre 5 et 70%.

4. Corps à libération pregressive de vapeurs selon la revendication 2 dans lequel la quantité de matériau polymère inorganique dans la couche de revêtement est de 4 fois en poids au moins de la quantité du matériau polymère formant la pellicule.

5. Corps à libération progressive de vapeurs selon la revendication 1 dans lequel le matériau polymère formant la pellicule est un dérivé de la cellulose soluble dans un solvant ayant un point d'ébullition de 120°C au moins.